# NEUE EUROPÄISCHE PATENTSCHRIFT

(11) **EP 1 235 532 B2**
(45) Veröffentlichungstag und Bekanntmachung des Hinweises auf die Entscheidung über den Einspruch: **10.04.2013**
(45) Hinweis auf die Patenterteilung: 25.05.2005
(21) Anmeldenummer: 00988789.4
(22) Anmeldetag: 07.12.2000
(51) Int. Cl.: A61C 13/00, A61K 6/06

(54) **VERFAHREN ZUR HERSTELLUNG KERAMISCHEN ZAHNERSATZES**
METHOD FOR PRODUCING A CERAMIC DENTAL PROSTHESIS
PROCEDE DE FABRICATION D'UNE PROTHESE DENTAIRE CERAMIQUE

(30) Priorität: 07.12.1999 DE 19958881
(43) Veröffentlichungstag der Anmeldung: 04.09.2002
(73) Patentinhaber: ce.novation GmbH, 07629 Hermsdorf (DE)
(72) Erfinder: LUTHARDT, Ralph, Gunnar, 01309 Dresden (DE); HEROLD, Volker, 07749 Jena (DE); JOHANNES, Martina, 07629 Hermsdorf (DE); SANDKUHL, Olaf, 07751 Cospeda (DE)
(74) Vertreter: Oehmke, Volker
(86) Internationale Anmeldenummer: PCT/EP2000/012599
(87) Internationale Veröffentlichungsnummer: WO 2001/041670

(56) Entgegenhaltungen:
- EP-A- 0 580 565
- EP-A- 0 943 296
- ROBERT T TOTH: 'CEREC 2, Vollkeramische CAD/CIM Computerkronen. Kostruktion im Front-und Seitenzahnbereich', 01 Juni 1999, STIFTUNG ZUR FÖRDERUNG DER COMPUTER-ZAHNMEDIZIN, ZÜRICH, ISBN 395217520X vol. WERNER H. MÖRMANN
- SIRONA DENTAL SYSTEMS GMBH & CO.KG: 'CROWN 1.0,Handbuch für den Anwender', Dezember 1997, SIRONA DENTAL SYSTEMS, DEUTSCHLAND

## Beschreibung

Die Erfindung betrifft ein Verfahren zur Herstellung keramischen Zahnersatzes, wie beispielsweise Inlays, Onlays, Teilkronen, Kronen, Brücken und Implantat-Suprastrukturen. Keramik ist als Material für Zahnersatz aller Art wegen ihres Aussehens und ihrer Festigkeit, welche den Eigenschaften natürlicher Zähne nahekommen, in verschiedenen Ausführungsformen bekannt. Bei richtiger Wahl des Werkstoffes ist Keramik zugleich physiologisch unbedenklich. Dabei ist weniger das Material an sich als vielmehr die erforderliche genaue End-Formgebung ein beträchtlicher Kostenfaktor, wobei die gewünschte hohe Festigkeit des Zahnersatzes und eine Finish-Bearbeitbarkeit desselben im Zustand gebrannter Keramik einander zuwiderlaufen.

Es ist auch allgemein bekannt, Zahnersatz aus speziellen Metall-Legierungen zu gießen, wobei beispielsweise die einem Zahnstumpf entsprechende Innenform über einen Abdruck gewonnen wird. Diese Verfahrensweise ist auf gießbare Metall-Legierungen beschränkt.

Weiter ist es bekannt, ein zuvor gefertigtes Modell des Zahnersatzes zu kopieren, indem ein Taststift auf dem Modell entlanggeführt wird, durch welchen synchron zu dessen Bewegung eine Schleifscheibe bzw. ein Schleifkörper zur Bearbeitung eines Zahnersatzrohlings geführt wird (EP 0 267 227 B2). Prinzipiell sind dadurch Werkstoffe mit nahezu beliebiger Härte und Festigkeit, wie beispielsweise auch Keramik bearbeitbar. Dies erfordert jedoch einen sehr komplizierten Mechanismus, und dennoch sind der Bearbeitung von feinen Details, insbesondere im Falle von Vertiefungen und von Hinterschneidungen, durch die endlichen Abmessungen der Schleifscheibe bzw. des Schleifkörpers Grenzen gesetzt.

Ebenfalls bekannt sind CAD-CAM-Verfahren zum dreidimensionalne Fräsen hartgebrannten keramischen Vollmaterials zur Herstellung von Zahnersatz, bei denen zum Teil sogar patientenbezogene Informationen, wie Farbe, Material und Bißregistrierung, in den Rechner eingegeben und am Bildschirm bearbeitet werden sowie aus in einer Datenbank enthaltenen "Kronenmodellen" am Bildschirm eine Auswahl und "Modellierung" erfolgt (Aufsatz von A. Schmidt, M. Walter, K. Böning "CAD/CAM/CIM-Systeme in der restaurativen Zahnmedizin", Quintessenz 49, 11, S. 1111 - 1122 (1998)). Wenn auch hier gegenüber dem zuvor besprochenen Stand der Technik eine größere Formenvielfalt und größere Detailtreue möglich sind, ohne daß zwingend Modelle angefertigt werden müssen, so verbleibt als Nachteil doch der hohe Aufwand für die Bearbeitung harter (gebrannter) Keramik.

Harte Zahnersatzrohlinge lassen sich auch durch Erosionsverfahren, wie Ultraschall- oder Elektro-Erosion in die endgültige gewünschte Form bringen, wobei zuvor die Bearbeitungswerkzeuge, wie Sonotroden bzw. Elektro-Erosions-Elektroden, aus Abdrücken bzw. von Modellen als genaues komplementäres Abbild der gewünschten Form hergestellt werden. Das Verfahren der Elektro-Erosion ist prinzipiell auf elektrisch leitfähige Werkstoffe beschränkt, während die Ultraschall-Erosion diese Beschränkung nicht aufweist und ausdrücklich auch bei keramischen Werkstoffen angewandt wird. Durch eine Teilung entlang des sogenannten "Äquators" können sowohl die zervikale (dem Kiefer zugewandte) Seite als auch die okklusale bzw. die inzisale (der Mundhöhle zugewandte) Seite bearbeitet werden (EP 0 645 195 A1). Eine Kombination beider Erosions-Prinzipien gestattet nach einer ausgeklügelten Verfahrensweise die paßgenaue Herstellung eines keramischen Zahnersatzes oder auch einer Metallkrone, wobei durch galvanische Auftrágung auf zuvor gefertigte Modelle sogar ein Satz von Schrupp- (Grob-) und Feinbearbeitungswerkzeugen herstellbar ist und bisherige Verfälschungen der Paßgenauigkeit überwunden werden sollen (EP 0 614 344 B1). Der Verfahrensablauf wird danach differenziert, ob Keramik oder Metall bearbeitet werden soll, wobei die Anwendung der Funkenerosion in diesem Falle ein Formwerkzeug aus Graphit erfordert. Es bleiben als Nachteile dieser Verfahren die langen Bearbeitungszeiten und vor allem bei der Verfahrensweise nach der an zweiter Stelle genannten Veröffentlichung die Vielzahl verschiedener aufeinander folgender und aufeinander abzustimmender Verfahrensschritte.

Zur Herstellung sogenannter Jacket-Kronen ist es bekannt, eine zuvor gefertigte, flexible Keramikfolie im plastischen Rohzustand isostatisch auf ein Gipsmodell der Innenform zu pressen, welches zuvor computergestützt entsprechend der Form des Zahnstumpfes unter Berücksichtigung eines Aufmaßes für die Sinterschwindung der Keramik geformt wurde (EP 0 826 642 A1). Da die Folie in einer bestimmten Dicke hergestellt wird, welche bei der Umformung am Modell nur wenig verformt werden kann, ohne zu reißen, ist dieses Verfahren auf die Anfertigung von Kronen beschränkt und nicht auf andere Formen des Zahnersatzes übertragbar.

Schließlich ist es bekannt, die zervikale Form eines auf eine oder mehrere vorbereite Zahnstumpfflächen oder künstliche Halteteile aufzusetzenden Zahnersatzes, dessen okklusale Form mit aufgebranntem Dentalporzellan verblendet wird, durch Trockenpressen oder auch durch Schlickergießen auf einem Modell beispielsweise aus Gips herzustellen, welches zuvor mittels einer computergesteuerten Fräsmaschine entsprechend einer dreidimensionalen Abtastung direkt im Munde oder an einem Abgußmodell unter der späteren Sinterschwindung und eines Spaltes für den Zement zu Befestigung hergestellt worden war (EP 0 580 565 B1 entspricht DE 693 20 563 T2, WO 94/27 517 A1). Bezüglich der okklusalen Form des Zahnersatzes ist lediglich gesagt, daß die äußere Oberfläche des auf diese Weise hergestellten Kernes des Zahnersatzes eine Form "nahe der erwünschten Größe" haben soll, die Kauflächen nach diesem Verfahren also nicht direkt hertsellbar sind, sondern einer Nachbearbeitung im gebrannten Zustand bedürfen. Dazu sind jedenfalls weitere Verfahrensschritte mit mindestens den gleichen Anforderungen an Paßgenauigkeit sowie an Aussehen und Detailtreue erforderlich, welche den Bearbeitungsaufwand insgesamt zumindest verdoppeln. Das Trockenpressen von Teilen aus Hochleistungskeramik weist den Nachteil ungleichmäßiger Verdichtung bei der Formgebung auf, wodurch nach dem Brennen bzw. Sintern Defekte in Gestalt eines teilweise porösen Gefüges zurückbleiben.

Eine weitere bekannte, sehr komplizierte und materialaufwendige Verfahrensweise zur Herstellung keramischen Zahnersatzes soll die eingangs geschilderte und insbesondere wegen des Werkzeugverschleißes problematische End-Formgebung im fertiggesinterten (harten) Zustand vermeiden und zugleich das Problem lösen, daß dünne Randbereiche des Zahnersatzes sich beim Sintern infolge der Sinterschrumpfung verziehen oder brüchig werden. Dazu werden die negative Außen- und Innenform des Zahnersatzes aus einem kaltverdichteten oder teilgesinterten Form-Rohling unter Berücksichtigung der Sinterschwindung herausgearbeitet und aus den Bearbeitungsspänen oder einem gleichartigen Material der Zahnersatz in dieser, noch mit einem Trennmittel behandelten Form geformt und fertig gesintert (WO 96/29951 A2). Dabei kann auch auf die Herstellung eines Wachs- oder Kunststoffmodelles verzichtet werden, indem die Inlay-Außenform sowie die Kavitätsform bzw. die Form des präparierten Zahnstumpfes für die computergestützte Herstellung der Form direkt durch Abtastung im Mund des Patienten erfaßt werden. Die Mängel dieser technischen Lösung liegen sicherlich nicht in der Qualität des solcherart hergestellten Zahnersatzes sondern in der langen Dauer des Herstellungsverfahrens sowie im dazu erforderlichen Aufwand.

Es ist ferner bekannt, ein Positivmodell des Grundgerüstes für die aufzusetzenden Zahnkronen und/oder Zahnbrücken herzustellen, welches abgetastet und digitalisiert sowie zur Kompensation der Sinterschwindung in allen Raumrichtungen vergrößert wird. Dieses vergrößerte Modell wird dann einer elektronisch gesteuerten Bearbeitungsmaschine zugeführt, welche einen Rohling aus poröser (teilgesinterter) Keramik bearbeitet, der dann fertiggebrannt wird. Dabei ist es auch möglich, fehlende Teile des Positivmodelis zur Erstellung von Zahnbrücken rechentechnisch zu ergänzen (EP 0943 296 A1). Der Nachteil dieser technischen Lösung besteht in der erforderlichen allseitigen Bearbeitung des porösen Rohlings, was einen hohen Aufwand der mechanischen Endbearbeitung auch hinsichtlich der erforderlichen Werkzeuge aus Hartstoffen verursacht und trotz der Einrechnung der Sinterschwindung beim Fertigsintern noch Unsicherheiten bezüglich der Sinter-Vorgeschichte mit möglichen Texturen aus derselben birgt.

Schließlich ist es bekannt, die dem Zahnstumpf bzw. dem Kiefer zugewandte Innenform von Zahnersatz durch ein bestimmtess Urformungsverfahren, nämlich durch das (Trocken-)Pressen eines Keramikpulvers herzustellen, wobei ebenfalls eine optische oder mechanische Abtastung für den Zahnersatz vorbereitenen Bereiche entweder direkt im Mund des Patienten oder auf einem Positivmodell davon erfolgt und bei einem Kopierfräsvorgang einer Negativform für die o.g. Formung dann eine Vergrößerung zur Berücksichtigung der Sinterschwindung sowie eines Zementspaltes erfolgt. Jedoch wird die der Mundhöhle zugewandte Außenform des Zahnersatzes durch Aufsetzen gesonderter Teile geschaffen, über deren Formgebung nichts gesagt wird (EP 0 580 565 B1). Nachteilig sind bei dieser technischen Lösung neben der komplizierten und damit teuren Verfahrensweise, die meßtechnischen Probleme der Überbestimmung und der Kettenmaßtolerierung beim Zusammenfügen der Teile für Innen- und Außenform sowie zumindest beim Trockenpressen, das bei dieser technischen Lösung im Hauptanspruch verankert ist und damit zwingend anzuwenden ist, die Notwendigkeit, eine bestimmte Mindest-Wanddicke einzuhalten, so daß das Problem bleibt, den allmählich dünner werdenden Restaurationsrand durch die oben erwähnten, gesondert aufgesetzten Teile zu realisieren.

Die im vorigen Absatz genannte Veröffentlichung erwähnt auch hochfeste Aluminiumoxidkeramik, jedoch nur für die zuvor beschriebene Innenform, nicht für die Außenform.

Zahnersatz aus Aluminiumoxid, ggf. mit Zusatzstoffen, sowie aus Zirkonoxid ist unabhängig von einem bestimmten Herstellungsverfahren desselben bekannt (EP 0 593 611 B1).

Ausgehend von dem eingangs beschriebenen Stand der Technik liegt der vorliegenden Erfindung nun die Aufgabe zu Grunde, ein Verfahren zur Herstellung von Zahnersatz, insbesondere von festsitzendem keramischen Zahnersatz, zu schaffen, bei welchem unter Vermeidung der Hartbearbeitung der Keramik und mit einem Minimum an Verfahrensschritten, die der Mundhöhle zugewandte/Außenform keramischen Zahnersatzes einschließlich des allmählich dünner werdenden Restaurationsrandes in ihrer endgültigen Form gefertigt wird, wobei der Sinterschwindung Rechnung getragen werden soll.

Diese Aufgabe wird erfindungsgemäß, mit den Verfahren gemäß Anspruch 1 gelöst.

Je nach der Form des zu fertigenden Zahnersatzes werden ein- oder mehrteilige Formen zur Anwendung kommen. Vorzugsweise werden einteilige Formen bei hinterschnittfreien Geometrien des zu fertigenden Zahnersatzes angewendet werden. Bei komplexeren Geometrien des zu fertigenden Zahnersatzes werden vorzugsweise zwei- oder ggf. mehrteilige Formen zu Anwendung kommen. Hinterschnittene Formen ergeben sich zwangsläufig bei der Fertigung von Teilkronen, Kronen oder Zahnersatz zur Rekonstruktion der funktionellen Kauflächen und/ oder Brücken und/ oder Implantatsuprastrukturen. Trennebenen verlaufen dabei vorzugsweise im Bereich des anatomischen Äquators des Zahnersatzes.

Durch die Geometrie der Form wird bei einer mehrteiligen Form die Außen- und auch Innenform des Zahnersatzes definiert. Hierbei wird die Außenform des Zahnersatzes als Negativ bearbeitet, während die Innenform des Zahnersatzes als Positiv bearbeitet wird. Bei Kronen oder Teilkronen oder Inlays werden vorzugsweise mit der Innenform auch die zervicalen Anteile des Restaurationen definiert werden. Bei Brücken werden zusammen mit der Innenform der Brücke vorzugsweise auch die Basalfläche des Brückenzwischengliedes definiert werden. Die dreidimensionale Zuordnung der Teilformen zueinander erfolgt vorzugsweise über Bestimmungs- und Spannflächen.

Durch die Konstruktion der Form des Zahnersatzes, die vorzugsweise durch CAD/CAM-Technologien erfolgt, ist es möglich die dreidimensionalen Dimensionsänderungen, die bei der Urformung auftreten, rechnergestützt für jede Restauration zu simulieren und zu berechnen. Als Grundlage der dentalen CAD/CAM-Technologien dienen Digitalisierdaten der präparierten Zähne, die durch intraorale oder extraorale optische oder mechanische Vermessung von Zähnen, Modellen oder Teilmodellen eines oder beider Kiefer mit oder ohne die Erfassung der räumlichen Zuordnung von Ober- und Unterkiefer gewonnen werden. Die Digitalisierdaten der Nachbarzähne werden für die Gestaltung der approximalen Anteile der zu fertigenden Inlays, Onlays, Teilkronen, Kronen, Brücken und Implantatsuprastrukturen bzw. der Gerüste aller vorgenannten Restaurationen sowie Digitalisierdaten der antagonistischen Zähne in die Rekonstruktion der funktionellen Kauflächen der zu fertigenden Restaurationen genutzt. Die Qualität der Rekonstruktion der Kauflächen der Inlays, Onlays, Teilkronen, Kronen, Brücken und Implantatsuprastrukturen bzw. der Gerüste aller vorgenannten Restaurationen kann durch die Nutzung der Digitalisierdaten von Ober- und Unterkiefer zusammen mit Daten zu den Bewegungsbahnen der Kiefergelenke optimiert werden.

In die Simulation der dreidimensionalen Dimensionsänderungen der Werkstoffe während der Urformung fließen sowohl die Daten der präparierten Zähne, die Geometrie der konstruierten Inlays, Onlays, Teilkronen, Kronen, Brücken und Implantatsuprastrukturen bzw. der Gerüste aller vorgenannten Restaurationen, die Schichtstärken zahnärztlicher Befestigungswerkstoffe als auch technische Parameter des Formenwerkstoffes, der Keramik und weitere verfahrenstechnische Einflußgrößen des jeweiligen Urformverfahrens ein. Einteilige Formen werden dabei vorzugsweise aus Blockmaterialien hergestellt. Für komplexere Geometrien oder bei hinterschnittenen Außenformen des zu fertigenden Zahnersatzes ist eine Trennung der Form in zwei oder mehrere Teile notwendig. Hierfür kommen speziell vorgefertigte Rohlinge für Teilformen, die vorzugsweise die Trennebenen und Einguß- oder Einspritzkanäle für das jeweilige Formgebungsverfahren berücksichtigen zu Anwendung. Die einzelnen Teilformen werden dabei durch Bestimmungs- und Spannflächen eineindeutig einander zugeordnet. Die Bearbeitung der Teilformen erfolgt dabei gemeinsam oder auch getrennt vorzugsweise mit 3- oder mehrachsigen Bearbeitungsmaschinen.

Als Werkstoffe für die Herstellung des Zahnersatzes werden vorzugsweise Keramik, insbesondere hochfeste, hochreine Keramik (Al₂O₃, teil- oder vollstabilisiertes ZrO₂, Dispersionskeramik) mit mittleren Komgrößen im Gefüge < 1µm, aber auch Metalle eingesetzt. Zur Verbesserung der ästhetischen Erscheinung des Zahnersatzes werden sowohl hochfeste transparente oder entsprechend der 16 sogenannten "Vita-Farben" einfärbte Keramik eingesetzt. Der Einsatz von Gradientenwerkstoffen zur Optimierung der optischen und/ oder mechanischen Eigenschaften ist möglich. Die Möglichkeit der ästhetischen Optimierung der zu fertigenden Restaurationen erfolgt vorzugsweise mittels Dotanten im ppm-Bereich.

Die Urformung der Inlays, Onlays, Teilkronen, Kronen, Brücken und Implantatsuprastrukturen bzw. der Gerüste aller vorgenannten Restaurationen erfolgt vorzugsweise durch keramisches Schlickergießen oder keramischen Spritzguß.

Durch die Anwendung der Erfindung ist eine prinzipiell kostengünstigere Formgebung des Zahnersatzes möglich, indem die spanende Formgebung von der Hartbearbeitung der Keramik auf den wesentlich weicheren Werkstoff der Form zur Urformung, wie beispielsweise Gips, verlagert wird. Dabei ist zu bedenken, daß Zahnersatz mit dem Ziel der Rekonunstruktion der okklusalen (Kau-) Flächen insbesondere durch die vilefältige und komplexe Gestalt der Außenform gekennzeichnet ist, während die bisher bereits CAD-gestützt hergestellte Innenform relativ einfach ist.

Der Zahnersatz wird unter Erhalt seiner werkstoffinhärenten Festigkeit ("as fired") realisiert.

Durch die Anwendung der Erfindung wird die Herstellung metallfreien Zahnersatzes, wie beispielsweise Inlays, Onlays, Teilkronen, Kronen und und Implantat-Suprastrukturen sowie von Brücken mit funktionellen Kauflächen möglich, welche ohne weitere Nachbearbeitung die ästhetischen und biologischen Anforderungen an die definitive prothetische Versorgung erfüllen.

Weitere Einzelheiten und Merkmale des erfindungsgemäßen Verfahrens ergeben sich aus der nachfolgenden Beschreibung von Ausführungsbeispielen und anhand der Zeichnungen. Die einzelnen Figuren der Zeichnungen zeigen:
- Fig. 1:: eine Darstellung der prinzipiellen Vorgehensweise bei der Herstellung von Zahnersatz gemäß des erfindungsgemäßen Verfahrens,
- Fig. 2:: eine Darstellung einer einteiligen Gußform entsprechend dem erfindungsgemäßen Verfahren mit einer Außenform eines Kronengerüstes,
- Fig. 3:: eine Darstellung einer Gußform entsprechend dem erfindungsgemäßen Verfahren mit einer Innenform des Kronengerüstes,
- Fig. 4:: eine perspektivische Darstellung einer zweiteiligen Gußform entsprechend dem erfindungsgemäßen Verfahren,
- Fig. 5:: eine perspektivische Darstellung einer Gußform entsprechend dem erfindungsgemäßen Verfahren mit einer Außenform einer Krone mit funktioneller Kaufläche,
- Fig. 6:: eine Darstellung einer Gußform entsprechend dem erfindungsgemäßen Verfahren mit einer Innenform einer Krone,
- Fig. 7:: eine Darstellung einer zweiteiligen Gußform einer Krone mit funktioneller Kaufläche entsprechend dem erfindungsgemäßen Verfahren,
- Fig. 8:: eine Darstellung einer zweiteiligen Gußform eines Brückengerüstes.

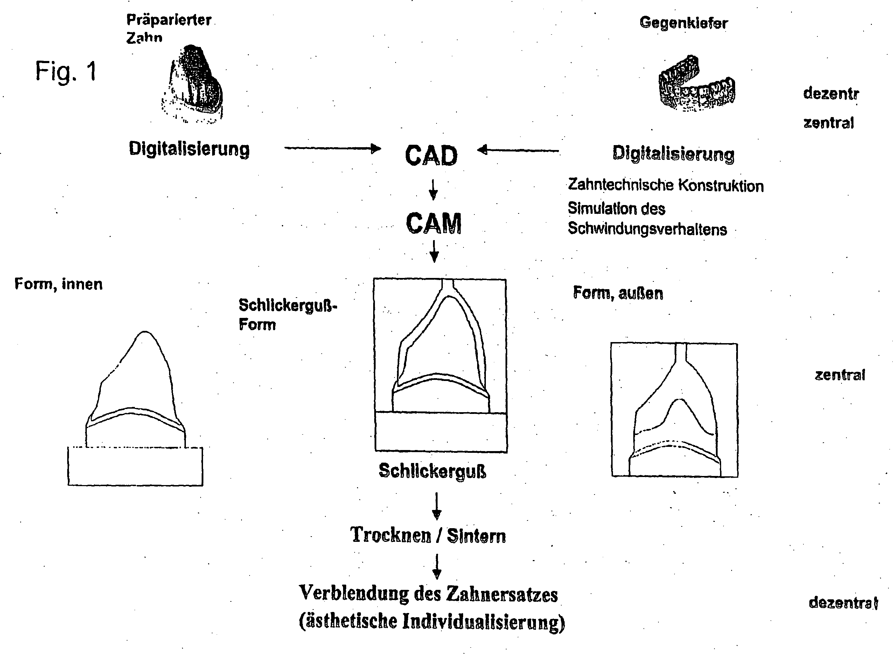

Die Kette der erfindungsgemäßen Verfahrensschritte (Fig. 1) ermöglicht eine kostenmäßige Optimierung durch eine Aufteilung in solche Verfahrensschritte, die zentral und solche, die dezentral durchzuführen sind. Für mehere Auftraggeber tätige CAD-CAM-Dienstleister bzw. spezialisierte Zahntechniker kooperieren beispielsweise in der folgenden günstigen, aber naturgemäß nicht zwingend vorgeschriebenen, Aufgliederung der Arbeitsgänge:
- Herkömmliche dezentrale zahnärztliche Behandlung mit Präparation
- Abformung und Herstellung von Präzisionsmodellen und deren optische Digitalisierung bzw. direkte intraorale Digitalisierung.
- Zentrale Berechnung der geometrischen Gestalt der Form für das Schlickergießen unter Berücksichtigung des simulierten Schwindungsverhaltens.
- Zentrale Erstellung des CNC-Programmes zur Bearbeitung der individuellen Form für das Schlickergießen sowie Herstellung derselben.
- Zentrales Gießen und Sintern des Zahnersatzes.
- Erforderlichenfalls dezentrale ästhetische Indiviualisierung des Zahnersatzes.

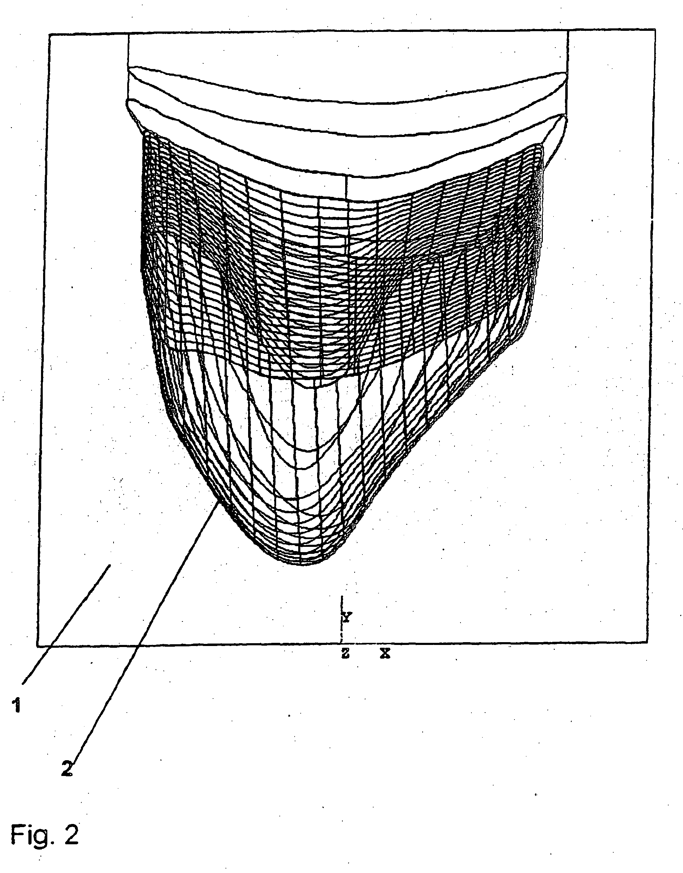

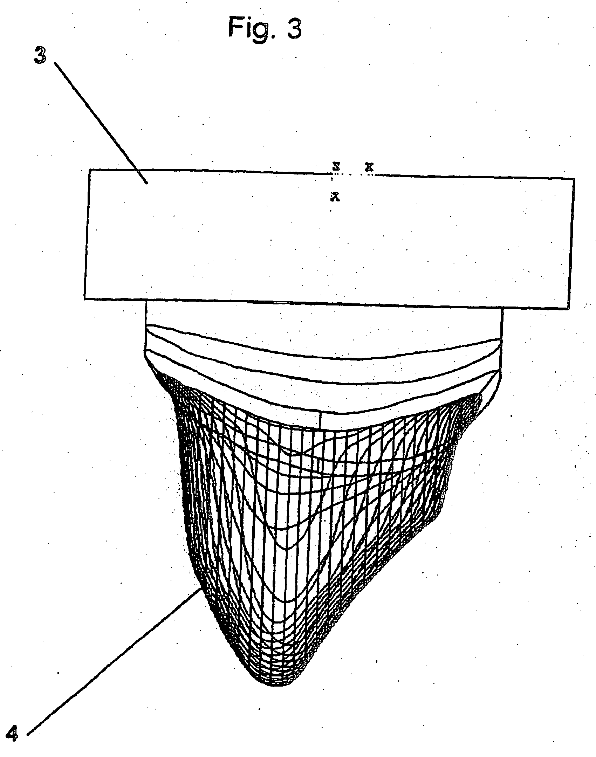

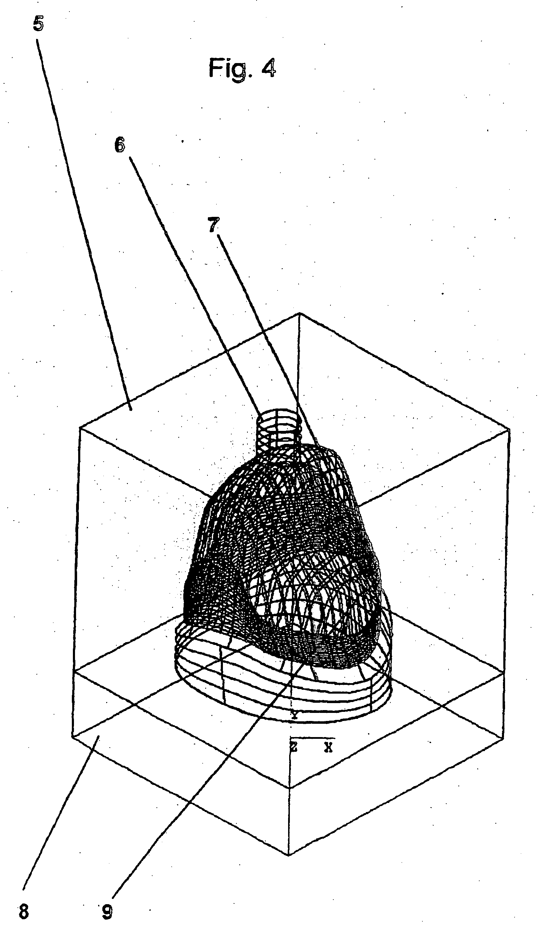

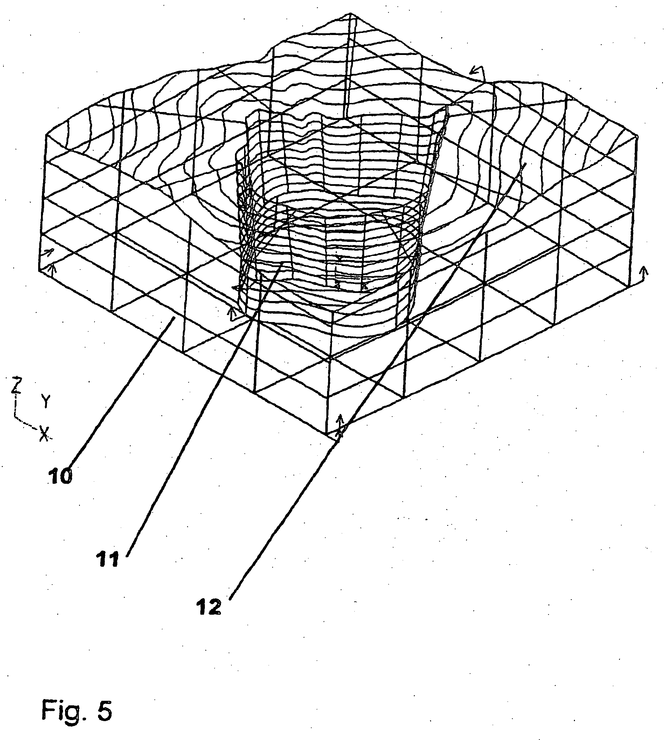

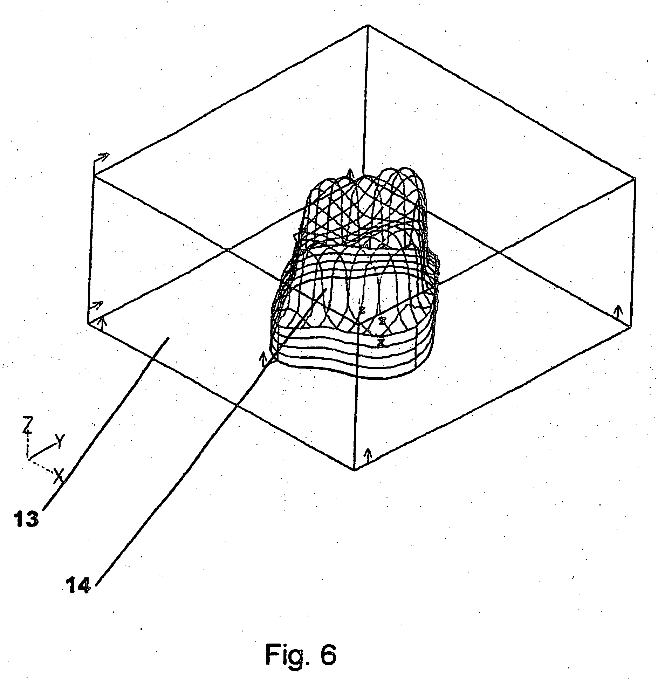

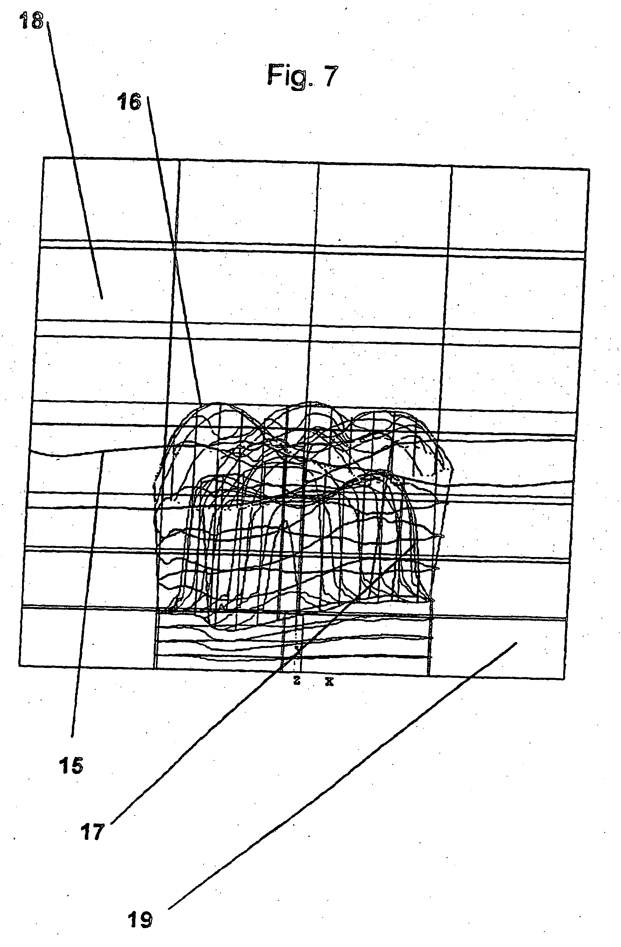

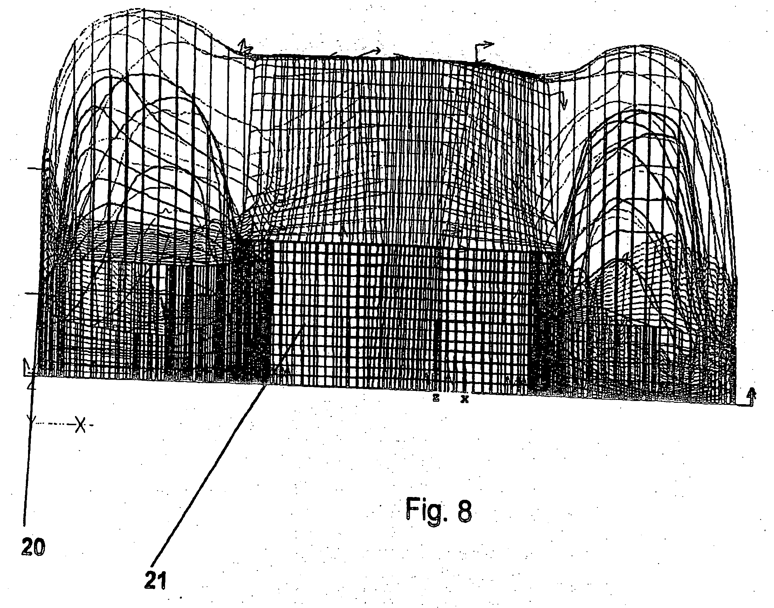

Die Formen, wie sie in den Figuren 2 bis 7 jeweils mit den X-, Y- und Z-Achsen ihres Systems der Bestimmungs- und Spannflächen dargestellt sind, dienen zur Umsetzung des Verfahrens zur Herstellung keramischen Zahnersatzes sowie danach hergestelltem hochfesten keramischen Zahnersatz. Diese Formen setzen sich zusammen aus der einteiligen oder mehrteiligen Form (Fig. 3), bestehend aus der äußeren Formhälfte 1 bzw. der äußeren Formhälfte 5, 10, 18 und der inneren Formhälfte 3, 8, 13, 19 zur Definition der Außenform 2 bzw. der Außenform 7, 11, 16, 20 und der Innenform 9, 14, 17, 21 der zu fertigenden Restaurationen, dem System der Gußkanäle 6 sowie den Trennflächen 12 bzw. 15. Fig. 2 zeigt eine hinterschnittfreie Außenform, welche prinzipiell eine einteilige Form zur Definition der Außenform 1 der zu fertigenden Restauration zulässt. Zusammen mit der in Fig. 3 dargestellten inneren Formhälfte 3 zur Definition der Innenform 4 der Restauration, können diese zu einer mehrteiligen Form (Fig. 4) zusammengesetzt werden. Fig. 4 zeigt weiterhin den Gußkanal 6 sowie die Trennebene. Mit zunehmender Komplexität der Geometrie zu fertigenden Restauration (Fig. 5, 6, 7) resultieren komplexere Trennflächen 12 bzw. 15. Fig. 8 zeigt die Außen- und Innenform (20 bzw. 21) einer Brücke.

Die Erfindung wird weiterhin an Hand von zwei Ausführungsbeispielen bezüglich des Materials weiter erläutert:

### Ausführungsbeispiel 1:

500 g Aluminiumoxid mit einer Korngröße von d₅₀ = 0,2 µm werden zusammen mit 0,05 Masse% Magnesiumcarbonat, 1,5 Masse% eines zur Bereitung von Gießschlickern bekannten Verflüssigers sowie Wasser in eine Trommelmühle gegeben. Das Masseverhältnis von Mahlgut : Mahlkörpem beträgt 1 : 6. Nach einer Mahldauer von 24 Stunden wird der Schlicker entnommen. Sein Feststoffgehalt beträgt 71,0 Masse%. Dieser Schlicker wird zur Herstellung einer Krone in die nach dem Schema der Abbildung hergestellte Gießform gefüllt. Nach einer Standzeit von etwa 30 Minuten wird die Krone entformt und nach entsprechender Trocknung mit einer Haltezeit von 2 Stunden bei 1350 °C sintert.

### Ausführungsbeispiel 2:

500 eines Versatzes aus 80 Masse% Aluminiumoxid und 20 Masse% teilstabilisiertem Zirkonoxid mit einer Komgröße von d₅₀ = 0,3 µm werden analog Ausführungsbeispiel 1 verarbeitet, dem einzigen Unterschied, daß die Sinterung bei 1400 °C erfolgt.

### Aufstellung der verwendeten Bezugszahlen

- 1: Äußere Formhälfte
- 2: Außenform
- 3: Innere Formhälfte
- 4: Innenform
- 5: Äußere Formhälfte
- 6: Gußkanal
- 7: Außenform
- 8: Innere Formhälfte
- 9: Innenform
- 10: Äußere Formhälfte
- 11: Außenform
- 12: Trennfläche
- 13: Innere Formhälfte
- 14: Innenform
- 15: Trennfläche
- 16: Außenform
- 17: Innenform
- 18: Äußere Formhälfte
- 19: Innere Formhälfte
- 20: Außenform
- 21: Innenform

## Patentansprüche

1. Verfahren zur Herstellung von auf wenigstens einen vorpräparierten Zahnstumpf aufpassbaren künstlichen Zahnersatz, dessen Geometrie sich in eine durch die Grenzfläche zwischen präpariertem Zahnstumpf und Zahnersatz bestimmte innere Geometrie und eine äussere Geometrie unterteilen lasst, wobei die Daten für die Herstellung der inneren Geometrie durch Digitalisierung der Zahnstümpfe bereit gestellt werden, **gekennzeichnet dadurch, dass**
- die Daten für die äussere Geometrie durch Digitalisierung der Umgebung der präparierten Zahnstümpfe und streichung der antagonistischen Zähne ermittelt werden und
- alle digitalisierten Daten bei der Urformung des aus durchgehend der gleichen hochfesten Keramik bestehenden Zahnersatzes genutzt werden, um dessen endgültige Form durch sintern unter Berücksichtigung des Sinterschwindung zu fertigen.

2. Verfahren nach Anspruch 1, **dadurch gekennzeichnet, daß** durch Schlickergießen als Urformverfahren eine Reproduktion physiologischer, natürlicher Kauflächen mit entsprechender Fissurentiefe und -radien erfolgt.

3. Verfahren nach einem der bisherigen Ansprüche **dadurch gekennzeichnet, daß** die äussere Geometrie um den Anteil der Verblendkeramik reduziert wird.

4. Verfahren nach einem der bisherigen Ansprüche, **dadurch gekennzeichnet, daß** die hochfeste Keramik mittels Dotanten im ppm-Bereich mit dem Ziel der Simulation der Farbgebung des natürlichen Zahnes unterschiedlich eingefärbt wird.

5. Verfahren nach einem der bisherigen Ansprüche, **dadurch gekennzeichnet, daß** eine hochfeste Keramik mit transparenten Eigenschaften Anwendung findet.

6. Verfahren nach einem der bisherigen Ansprüche außer Anspruch 2 und von diesem abhängige Ansprüche, **dadurch gekennzeichnet, daß** die Urformung durch keramisches Spritzgießen erfolgt.

## Claims

1. A method of preparing an artificial dental prosthesis which can be fitted on at least one tooth stump prepared in advance, the geometry of said prosthesis being divisible into an internal geometry, defined by the interface between the prepared tooth stump and the dental prosthesis, and into an external geometry, with the data for establishing the internal geometry being provided by digitising the tooth stumps, **characterised in that**
- the data for the external geometry are obtained by digitising the vicinity of the prepared tooth stumps and of their antagonistic teeth,
and
- all digitised data are used in the primary shaping of the dental prosthesis, which is made entirely from the same high-strength ceramic material, to obtain its final shape by sintering, making allowance for shrinkage during sintering.

2. Method according to claim 1, **characterised in that**, using slip casting as the primary shaping method, reproduction of physiological, natural masticatory surfaces of suitable fissure depth and radii is effected.

3. Method according to any one of the preceding claims, **characterised in that** the external geometry is reduced by the proportion of veneer ceramics.

4. Method according to any one of the preceding claims, **characterised in that** the high-strength ceramic material is dyed differently by means of doping agents in the ppm range for the purpose of simulating the colouring of the natural tooth.

5. Method according to any one of the preceding claims, **characterised in that** a high-strength ceramic material having transparent properties is used.

6. Method according to any one of the preceding claims, except claim 2 and its dependent claims, **characterised in that** said primary shaping is effected by ceramic injection moulding.

## Revendications

1. Procédé de préparation d'une prothèse dentaire artificielle qui peut être adaptée à au moins un chicot dentaire préconditionné, la géométrie de ladite prothèse étant divisible en une géométrie intérieure, définie par l'interface entre le chicot dentaire conditionné et la prothèse dentaire, et une géométrie extérieure, les dates pour la préparation de la géométrie intérieure étant fournies par une numérisation desdits chicots dentaires, ledit procédé étant **caractérisé en ce que**
- les dates pour la géométrie extérieure sont obtenues par la numérisation du voisinage des chicots dentaires conditionnés et de leurs dents antagonistes,
et
- **en ce que** toutes les dates numérisées sont utilisées dans la première mise en forme de la prothèse dentaire, qui est constituée entièrement de la même matière céramique de haute résistance, pour obtenir sa forme finale par frittage, tenant compte du rétrécissement après frittage.

2. Procédé selon la revendication 1, **caractérisé en ce qu'**une reproduction des faces occlusales naturelles présentant des fissures de profondeur et de rayons appropriés est effectuée en utilisant la coulée en barbotine comme procédé de première mise en forme.

3. Procédé selon l'une quelconque des revendications précédentes, **caractérisé en ce que** la géométrie extérieure est réduite par le contenu en céramique revêtue.

4. Procédé selon l'une quelconque des revendications précédentes, **caractérisé en ce que** la matière céramique de haute résistance est colorisée de manières différentes par l'intermédiaire de dopants dans le domaine ppm, afin de simuler la coloration de la dente naturelle.

5. Procédé selon l'une quelconque des revendications précédentes, **caractérisé en ce que** l'on utilise une matière céramique de haute résistance et avec des caractéristiques transparentes.

6. Procédé selon l'une quelconque des revendications précédentes, à l'exception de la revendication 2 et de ses revendications dépendantes, **caractérisé en ce que** ladite première mise en forme s'effectue par moulage par injection céramique.
